# EUROPEAN PATENT SPECIFICATION

(11) **EP 1 804 006 B1**
(45) Date of publication and mention of the grant of the patent: **23.06.2010**
(21) Application number: 06026941.2
(22) Date of filing: 27.12.2006
(51) Int. Cl.: F24F 3/16, B01D 53/14

(54) **Air filtering apparatus**
Luftfilterungsvorrichtung
Appareil de filtrage d'air

(30) Priority: 28.12.2005 JP 2005377732; 31.01.2006 JP 2006021796
(43) Date of publication of application: 04.07.2007
(73) Proprietor: SANYO ELECTRIC CO., LTD., Moriguchi-shi Osaka 570-8667 (JP)
(72) Inventor: Fukushima, Toshio, Ota-shi, Gunma 373-0813 (JP); Arakawa, Toru, Gunma 374-0066 (JP); Usui, Hiroaki, Ora-gun, Gunma 370-0533 (JP); Tamura, Takaaki, Ora-gun, Gunma 370-0533 (JP); Koyama, Koji, Ota-shi, Gunma 373-0815 (JP); Mizuma, Masato, Ota-shi, Gunma 373-0812 (JP); Uchida, Yoichi, Tochigi 326-0006 (JP); Takahashi, Kazuo, Gunma 373-0036 (JP)
(74) Representative: Glawe, Delfs, Moll

(56) References cited:
- WO-A-2004/008034
- WO-A-2005/012802
- JP-A- 2000 334 240
- JP-A- 2002 181 358
- US-A1- 2004 045 909

## Description

### BACKGROUND OF THE INVENTION

### 1. Field of the Invention

The present invention relates to an air filtering apparatus that can remove (inactivate, sterilize, etc.) microorganisms such as virus, bacteria, fungus, etc. floating in the air, for example, and particularly relates to a portable floor-mount type air filtering apparatus.

### 2. Description of the Related Art

For the purpose of removing microorganisms such as virus, bacteria, fungus, etc. floating in the air, there has beenproposed a sterilizing apparatus in which electrolytic water mist is diffused into the air so that the electrolytic water mist is brought into direct contact with microorganisms floating in the air, thereby inactivating the microorganisms such as virus, bacterial, fungus, etc. (for example, see JP-A-2002-181358).

The above-described sterilizing apparatus works effectively under a use environment which electrolytic water mist of mine particles easily reach, that is, in a relatively small space, however, it does not work effectively in a user environment which the electrolytic water mist hardly reaches, that is in a large space such as a kindergarten, an elementary/junior/high school, long-term care insurance facilities, a hospital or the like.

In order to solve the above problem, there has been proposed an air filtering apparatus in which a gas-liquid contact member having water retentivity is provided in a housing, indoor air is blown out to the gas-liquid contact member by an air blowing fan such as a sirocco fan or the like while electrolytic water achieved by electrolyzing tap water is dropped to the gas-liquid contact member, and then the indoor air is discharged to a large space.

In this device, microorganisms such as virus, bacterial, fungus, etc. floating in the air are inactivated by using active oxygen species such as hypochlorous acid, etc. contained in electrolytic water. The active oxygen species such as hypochlorous acid, etc. also react with plastic, metal, etc. and thus they are consumed by the reaction. Accordingly, it has been required that consumption of active oxygen specifies such as hypochlorous acid, etc. contained in electrolytic water before they are brought into contact with air is suppressed to the minimum level.

An other air filtering apparatus according to the preamble of claim 1 is disclosed in the WO 2004/008034 A1, in which a deodorizing solution is used for a purification cycle to illuminate pollutants, such as fine particulates, cigarette smoke and kitchen smells etc., out of the air of a room.

### SUMMARY OF THE INVENTION

The present invention has been implemented in view of the foregoing situation, and has an object to provide an air filtering apparatus that can perform efficient sterile filtration on air by prevent consumption of active oxygen specifies contained in electrolytic water as completely as possible before the active oxygen specifies are brought into contact with air.

Furthermore, another object of the present invention is to provide an air filtering apparatus that can perform more efficient sterile filtration on air by substantially uniformly dropping electrolytic water to a gas-liquid contact member.

This objects are achieved by an air filtering apparatus as defined in claim 1; the dependent claims are related to further developments of the present invention.

This air filtering apparatus according to the present invention is characterized by comprising:
a housing (2);
at least one partition plate (51, 52) for partitioning the inside of the housing into at least two chambers (60, 61, 62);
a gas-liquid contact member having water retentivity (5, 55);
an air blowing fan (7) for blowing air to the gas-liquid contact member so that the air is brought into contact with the electrolytic water flowing along the gas-liquid contact member;
a support tray (10) for receiving electrolytic water flowing out from the gas-liquid contact member;
a circulating pump (13) for pumping up the electrolytic water stocked in the support tray (10);
an electrolytic water generating unit (3) for electrolyzing water pumped up by the circulating pump to generate electrolytic water; and
an electrolytic water supply pipe (17) for supplying the gas-liquid contact member with the electrolytic water generated by the electrolytic water generating unit, wherein the gas-liquid contact member has a water spray portion (74) and the electrolytic water supply pipe (17) is connected to the water spray portion (74) .

According to this construction, the electrolytic water is fed through the support tray and the circulating pump into the electrolytic water generating unit. In the electrolytic water generating unit, the electrolytic water from the support tray (added with tap water or the like as occasion demands) is newly electrolyzed to generate electrolytic water. The electrolytic water concerned flows through the electrolytic water supply pipe into the water spray portion containing a water spray box (74') of the gas-liquid contact member, sprayed and infiltrated into the gas-liquid contact member. Accordingly, the electrolytic water infiltrating into the gas-liquid contact member is electrolytic water that is generated by the electrolytic water generating unit just before the electrolytic water infiltrates into the gas-liquid contact member. Accordingly, the consumption of active oxygen specifies contained in electrolytic water can be suppressed before the electrolytic water is brought into contact with air.

In this case, the electrolytic water supply pipe may be inserted in the water spray box of the gas-liquid contact member, andpluralwatersprayholes (17B) maybe formed in the electrolytic water supplypipe so as to be spaced from one another substantially at equal intervals. Furthermore, the water discharge amount of the circulating pump may be set to be larger than the water spray amount in the water spray box. In this construction, electrolytic water is sprayed from the plural water spray holes of the electrolytic water supply pipe, and thus the electrolytic water can be substantially uniformly infiltrated into the whole area of the gas-liquid contact member. When the discharge amount of the circulating pump is set to be larger than the water spray amount in the water spray box, the feeding power of the electrolytic water under pressure is increased, and thus the electrolytic water can be deeply pressure-fed to the back side.

Furthermore, the electrolytic water may be achieved by supplying current to electrodes to electrolyze tap water and contain active oxygen specifies. The active oxygen specifies may contain at least one material of hypochlorous acid, ozone and hydrogen peroxide. Furthermore, the polarities of the electrodes may be inverted periodically or irregularly under a predetermined condition.

Still furthermore, the gas-liquid contact member may be designed in a rectangular shape, and disposed so as to be tilted in the housing. Electrolytic water may be dropped from the upper side through the electrolytic water supply pipe to the upper edge portion of the tilted gas-liquid contact member, and air may be blown to the gas-liquid contact member in which the electrolytic water infiltrates, and then blown out to a room. The water spray holes formed in the electrolytic water supply pipe may be formed so as to face substantially sideways (in the horizontal direction) so that the electrolytic water can be substantially uniformly supplied to the upper edge portion.

In this case, the electrolytic water supply pipe may be inserted into the water spray portion (74) formed in the longitudinal direction of the gas-liquid contact member, and the water spray holes may be formed so as to face the inner wall of the water spray portion. Furthermore, the water spray holes may be formed in the electrolytic water supply pipe so as to be spaced from one another substantially at equal intervals along the axial direction of the electrolytic water supply pipe.

Still furthermore, the water spray portion may be equipped with a distributing sheet for uniformly diffusing the electrolytic water dropped from the water spray holes, thereby supplying the electrolytic water to the upper end portion of the gas-liquid contact member uniformly. A guide member (79A) which is disposed so as to face each water spray hole and guide electrolytic water discharged from the water spray hole concerned to the distributing sheet may be provided in the water spray portion.

Still furthermore, the air blowing fan (7) and the gas-liquid contact member (5, 55) may be accommodated in one chamber (60) of the at least two chambers, and the support tray (10), the circulating pump (13), the electrolytic water generating unit (3) and the electrolytic water supply pipe (17) may be accommodated in the other chamber (62).

According to the present invention, the electrolytic water is passed through the support tray and the circulating pump to the electrolytic water generating unit. In the electrolytic water generating unit, electrolytic water is generated by electrolyzingwater from the support tray (the electrolytic water from the gas-liquid contact member and/or tap water from the water supply tank (11)). The electrolytic water thus generated passes through the electrolytic water supply pipe, and flows into the water spray box of the gas-liquid contact member to be sprayed and infiltrated into the gas-liquid contact member. Accordingly, the electrolytic water infiltrating into the gas-liquid contact member is electronic water generated by the electrolytic water generating unit just before it enters the gas-liquid contact member, and thus the consumption of active oxygen species contained in the electrolytic water can be suppressed before the electrolytic water is brought into contact with air.

Still furthermore, according to the present invention, the electrolytic water discharged from the water spray holes is substantially uniformly supplied to the upper edge portion of the gas-liquid contact member, and thus the electrolytic water can be infiltrated into the whole area of the gas-liquid contact member. Accordingly, the air filtering efficiency of air passing through the gas-liquid contact member can be enhanced.

### BRIEF DESCRIPTION OF THE DRAWINGS

Fig. 1 is a perspective view showing an embodiment of a floor-mount type air filtering apparatus according to the present invention;
Fig. 2 is a perspective view showing the internal construction of the floor-mount type air filtering apparatus of Fig. 1;
Fig. 3 is a longitudinally-sectional view of a housing;
Fig. 4 is a front view showing a gas-liquid contact member;
Figs. 5A to 5C are systematic diagrams showing a mechanism for dropping electrolytic water to a gas-liquid contact member, wherein Fig. 5A is a side view, Fig. 5B is a cross-sectional view showing a water spray box and Fig. 5C is a diagram showing the construction of an electrolytic bath;
Fig. 6 is a diagram showing air cleaning;
Fig. 7 is a perspective view showing the internal construction of the floor-mount type air filtering apparatus of Fig. 1 when the gas-liquid contact member 5 comprises plural element portions E;
Fig. 8 is a side view showing a mechanism for dropping electrolytic water when the gas-liquid contact member shown in Fig. 7 is used;
Fig. 9 is a perspective view showing the gas-liquid contact member shown in Fig. 7;
Fig. 10 is a longitudinally-sectional view showing the gas-liquid contact member shown in Fig. 9; and
Fig. 11 is a partially cross-sectional view of the gas-liquid contact member shown in Fig. 9.

### DETAILED DESCRIPTION OF PREFERRED EMBODIMENTS

A preferred embodiment according to the present invention will be described hereunder with reference to the accompanying drawings. In the following description, a floor-mount type air filtering apparatus is representatively used as an air filtering apparatus of the present invention. However, the present invention is not limited to the floor-mount type air filtering apparatus, and it may be applied to any type of air filtering apparatus such as a wall-suspended type, a ceiling-suspended type, a ceiling-cassette type or the like.

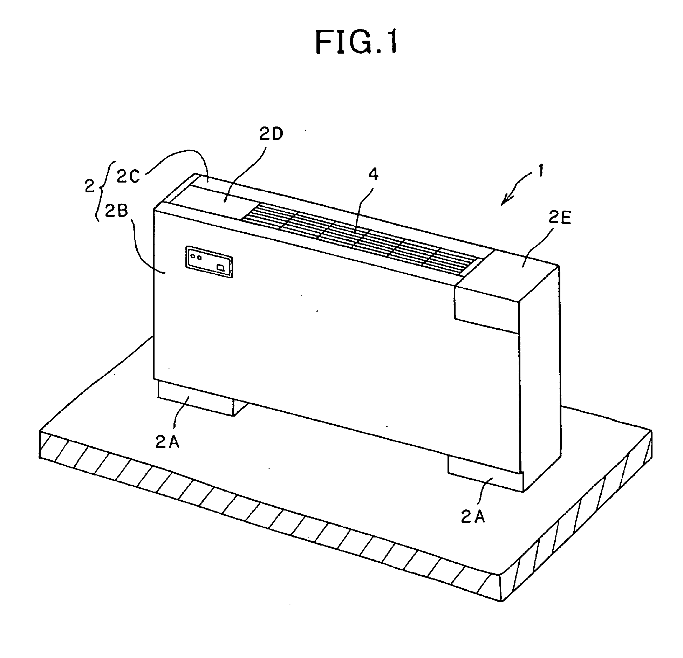

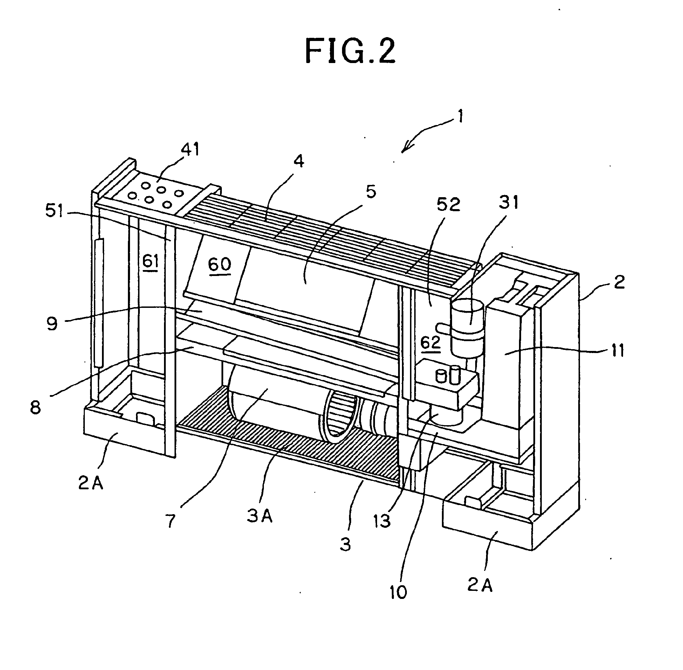

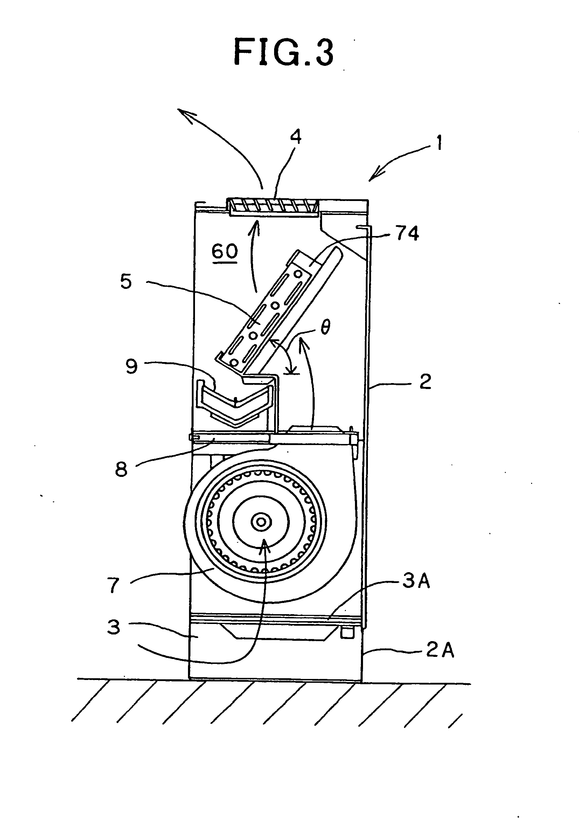

In Fig. 1, reference numeral 1 represents a floor-mount type air filtering apparatus. The floor-mount type air filtering apparatus 1 has a box-shaped housing 2, and the housing 2 has leg pieces 2A, a front panel 2B and a top panel 2C. An operation lid 2D and an opening/closing lid 2E are disposed in juxtaposition with each other. Furthermore, as shown in Fig. 2, a laterally elongated suction port 3 is formed at the lower portion of the housing 2, and a pre-filter 3A is disposed above the suction port 3. An air blowing fan 7 is disposed above the pre-filter 3A, and a gas-liquid contact member 5 having high water retentivity is disposed above the air blowing fan 7 so as to be tilted as shown in Fig. 3. Furthermore, a laterally elongated air blow-out port 4 is disposed above the gas-liquid contact member 5. Reference numeral 8 represents a support plate for the air blowing fan 7, and the support plate 8 is supported on the housing 2.

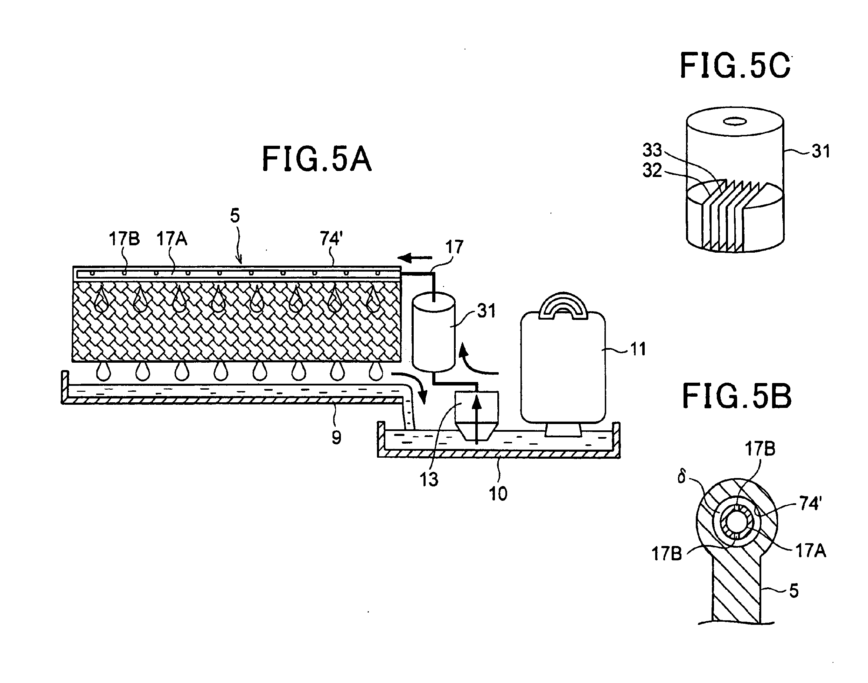

In Fig. 2, reference numerals 51 and 52 represent partition members (plates) for vertically partitioning the inside of the housing 2. The inside of the housing 2 is compartmented into three chambers (an air filtering chamber 60, an electrical component chamber 61 and a water supply chamber 62) by the partition plates 51 and 52. In the air filtering chamber 60 are disposed the gas-liquid contact member 5 having water retentivity, and the air blowing fan 7 for blowing indoor air to the gas-liquid contact member 5, and in the water supply chamber 62 are disposed a water supply tank support tray 10 (hereinafter referred to as "support tray" 10) for stocking electrolytic water flowing out from the gas-liquid contact member 5, a circulating pump 13 for pumping up electrolytic water stocked in the support tray 10, an electrolytic water generating unit (electrolytic bath) 31 for electrolyzing water pumped up by the circulating pump 13 to generate electrolytic water, and an electrolytic water supply pipe 17 (see Fig. 5) for supplying the gas-liquid contact member 5 with the electrolytic water generated in the electrolytic bath 31. Furthermore, in the electrical component chamber 61 is disposed a control board (not shown) for controlling the whole air filtering apparatus, etc. The gas-liquid contact member 5 is designed in a rectangular shape, and it is disposed in the hosing so as to be tilted (see Fig. 3) . Accordingly, the height and depth of the housing 2 can be reduced without reducing the contact area of the gas-liquid contact member 5 with air, and thus the housing 2 can be miniaturized.

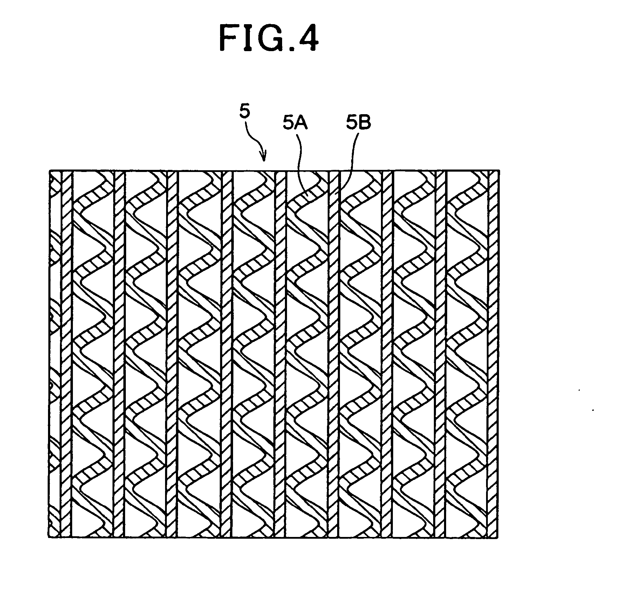

The gas-liquid contact member 5 comprises a filter member having a honeycomb structure, and it is designed so that a broad gas-liquid contact area can be secured, electrolytic water can be dropped and clogging occurs hardly. That is, as shown in Fig. 4, the gas-liquid contact member 5 comprises corrugated raw materials 5A and flat-plate type raw materials 5B joined to the corrugated raw materials 5A, and it is designed in a honeycomb structure as a whole. These raw materials 5A and 5B are formed of a material having little reactivity to electrolytic water described later, that is, a material which is hardly deteriorated by electrolytic water, such as a polyolefin resin group, a PET resin group, a vinyl chloride resin group, a fluorocarbon polymer group, a ceramic resin group or the like. The inclination (tilt) angle θ of the gas-liquid contact member 5 is preferably set to 30° or more. If the inclination angle θ is less than 30° , dropped electrolytic water does not flow along the slope of the gas-liquid contact member 5, but falls downwardly. On the other hand, if the inclination angle θ approaches to 90°, the air flow passage of the air passing through the gas-liquid contact member 5 is nearly horizontal, and thus it is more difficult to blow out the air upwardly. When the air blow-out direction concerned is set to the horizontal direction, it is impossible to blow out air to a remote place, and thus the apparatus is unsuitable for air filtering in a large space. Accordingly, it is preferable that the inclination angle θ satisfies 80°>θ>30°, and more preferably 55°<θ<75°. In this embodiment, the inclination angle θ is set to about 57°.

Figs. 5A to 5C show a mechanism for dropping electrolytic water to the gas-liquid contact member 5.

As shown in Fig. 3, a water receiving tray 9 is disposed below the gas-liquid contact member 5, and the water supply tank support tray (hereinafter referred to as "support tray") 10 intercommunicates with the water receiving tray 9 or the support tray 10 is disposed at the lower side of the water receiving tray 9. A water supply tank 11 for supplying water (tap water or the like) containing chlorine ions and the circulating pump 13 are disposed in the support tray 10. The electrolytic bath 31 is connected to the circulating pump 13, and a short plastic electrolytic water supplypipe 17 is connected to the electrolytic bath 31.

As shown in Figs. 5A and 5B, an insertion portion 17A of the electrolytic water supply pipe 17 is inserted in a water spray box 74' of a water spray portion 74 formed at the upper edge portion of the gas-liquid contact member 5 so as to be spaced from the inner wall of the upper edge portion at a predetermined interval δ. Many water spray holes 17B are formed on the outer peripheral portion of the insertion portion 17A so as to be spaced from one another at an equal interval in the axial (longitudinal) direction of the insertion portion 17A from the left side to the right side of Fig. 5A.

The total amount of water sprayed from the many water spray holes 17B is set to be smaller than the discharge amount of the circulating pump 13. Accordingly, according to this construction, the pressure of electrolytic water in the insertion portion 17A of the electrolytic water supplypipe 17 is increased, so that the electrolytic water is sufficiently supplied to all the water spray holes 17B from end to end. Accordingly, the electrolytic water can be evenly infiltrated to the gas-liquid contact member 5 from end to end in the longitudinal direction of the gas-liquid contact member 5.

As shown in Fig. 5C, the electrolytic bath 31 has plural pairs of electrodes 32 and 33. By supplying current to these electrodes 32 and 33, water flowing into the electrolytic bath 31 is electrolyzed to generate active oxygen species. Here, the active oxygen species are defined as molecules having higher oxidizing activity than normal oxygen molecules and relating materials, and contain so-called narrowly-defined active oxygen such as superoxide anion, singlet oxygen, hydroxyl radical, hydrogen peroxide, etc. and also so-called broadly-defined active oxygen such as ozone, hypohalous acid, hypochlorous acid, etc. The electrolytic bath 31 is disposed in proximity to the gas-liquid contact member 5, and it immediately supply the gas-liquid contact member 5 with active oxygen species generated by electrolyzing water pumped by the circulating pump 13.

Each of the electrodes 32 and 33 is an electrode plate comprising abase of Ti (titan) and a coating layer of Ir (iridium), Pt (platinum), and the current value applied to the electrodes 32, 33 is set to several mA to several tens mA (milliampere) /cm² (square centimeter) in current density to generate a predetermined free residual chlorine concentration (for example, 1mg (milligram)/l (liter)).

By supplying current to tap water through the electrodes 32, 33, at the cathode, the following reaction occurs:

4H⁺ + 4e⁻ + (4OH⁻) → 2H₂ + (4OH⁻)

At the anode, the following reaction occurs:

2H₂O → 4H⁺ + O₂ + 4e⁻

At the same time, chlorine ions contained in water (pre-added in tap water) make the following reaction:

2Cl⁻ → Cl2 + 2e⁻

Furthermore, Cl₂ reacts with water as follows:

Cl₂ + H₂O → HClO + HCl

In this construction, HClO (hypochlorous acid) having high sterilization power is generated by supplying current to the electrodes 32, 33, and various microorganisms such as bacteria, virus, etc. can be prevented from breeding in the gas-liquid crystal member 5 by making air pass through the gas-liquid contact member 5 to which electrolytic water containing hypochlorous acid is supplied. Therefore, microorganisms such as virus, bacteria, etc. floating in the air passing through the gas-liquid contact member 5 can be inactivated. Odor also reacts with hypochlorous acid in the electrolytic water when passing through the gas-liquid contact member 5, so that it is ionized and dissolved in the electrolytic water. Therefore, the odor can be removed from the air and thus the air is deodorized.

Next, the operation of this embodiment will be described.

In Fig. 1, when the operating lid 2D is opened, an operation panel (not shown) is exposed. By operating this operation panel, the operation of the floor-mount type air filtering apparatus 1 is started. When this operation is started, the circulating pump 13 is driven, and water (for example, tap water) stocked in the support tray 10 is supplied to the electrolytic bath 31.

In this electrolytic bath 31, the water is electrolyzed by supplying current to the electrodes 32 and 33 to generate electrolytic water containing active oxygen specifies. This electrolytic water flows out through the spray holes (not shown) of the electrolytic water supply pipe 17 to be sprayed into the water spray box 74'. The electrolytic water thus sprayed infiltrates into the upper edge portion of the gas-liquid contact member 5 and gradually diffuse to the lower portion of the gas-liquid contact member 5.

Surplus electrolytic water is collected in the water receiving tray 9, flows to the adjacent support tray 10 and then is stocked there. In this construction, the water supply system is a circulating system, and when the amount of water is reduced due to vaporization or the like, a suitable amount of water (tap water or the like) is supplemented through the water supply tank 11 into the support tray 10. When the amount of water in the water supply tank 11 is reduced, the opening/closing lid 2E (see Fig. 1) is opened, and the water supply tank 11 is taken out to supplement water (tap water or the like) into the water supply tank 11.

Indoor air is supplied through the air blowing fan 7 to the gas-liquid contact member 5 filled with the electrolytic water as indicated by an arrow X (see Fig. 3) . The indoor air is brought into contact with the active oxygen specifies of the electrolytic water infiltrating in the gas-liquid contact member 5 and then blown out to the room again. For example, when influenza virus inducing infection is floated in the indoor air, the active oxygen species has a function of breaking and vanishing (removing) the surface proteins of the virus. When influenza virus is broken, the influenza virus is not bonded to a receptor which is required for the influenza virus to infect, and thus the infection can be prevented. As a demonstration test result, it has been found that when air having influenza virus floated therein is passed through the gas-liquid contact member 5 of this embodiment, 99% or more of influenza virus can be removed (inactivated).

In this embodiment, as described above, the electrolytic water is passed through the support tray 10 and the circulating pump 13 and then fed to the electrolytic bath 31. In the electrolytic bath 31, water such as tap water is electrolyzed to generate electrolytic water, and the electrolytic water thus generates passes through the electrolytic water supply pipe 17, and directly flows into the water spray box 74' of the gas-liquid contact member 5. Then, the electrolytic water is sprayed and gradually infiltrated into the gas-liquid contact member 5. In the air filtering apparatus of this embodiment, microorganisms such as virus, bacterial, fungus, etc. are inactivated by using active oxygen specifies such as hypochlorous acid, etc. contained in the electrolytic water, and the active oxygen specifies concerned also react with plastics, metal, etc. and thus they are consumed by these materials. However, according to this embodiment, the electrolytic water to be infiltrated into the gas-liquid contact member 5 is generated by electrolysis in the electrolytic bath 31 just before the electrolytic water is infiltrated into the gas-liquid contact member 5, and also only the short plastic electrolytic water supplypipe 17 exists between the gas-liquid contact member 5 and the electrolytic bath 31. Therefore, the consumption of the active oxygen specifies by plastics, metal, etc. can be suppressed, and electrolytic water having a sufficient amount of oxygen active specifies can be supplied to the gas-liquid contact member 5.

According to the above construction, indoor air sucked from the suction port 3 provided at the lower portion of the housing 2 is brought into contact with the electrolytic water dropped to the gas-liquid contact member 5, and then the indoor air is blown out from the blow-out port 4 provided at the upper portion of the housing 2. Therefore, even when the floor-mount type air filtering apparatus 1 is put in a so-called large space such as a kindergarten, an elementary/junior/high school, long-term care insurance facilities, a hospital or the like, the indoor air which has been brought into contact with the electrolytic water and thus subjected to sterile filtration can be blown out to a remote place in a large space, and thus the air filtering in a large space can be efficiently performed.

Furthermore, the gas-liquid contact member 5 is disposed in the floor-mount type housing 2 so as to be tilted. Therefore, as compared with a case where the gas-liquid contact member 5 is disposed in a horizontal position, the depth dimension of the housing 2 can be reduced and thus the housing 2 can be designed to be thin. Furthermore, the gas-liquid contact member 5 is formed of a raw material having little reactivity with electrolytic water. Therefore, the durability and lifetime of the gas-liquid contact member 5 can be enhanced. Furthermore, the electrolytic water is supplied in a drop style, and thus the electrolytic water can be uniformly infiltrated into the whole area of the gas-liquid contact member 5, so that air passing through the gas-liquid contact member 5 canbe subjected to sterile filtration from end to end.

according to this embodiment, electrolytic water containing hypochlorous acid, etc. is collected in the water receiving tray 9, and flows to the adjacent support tray 10. Therefore, no microorganism occurs in each tray and thus occurrence of slime is prevented. Accordingly, the cleaning and maintenance frequency of each tray can be reduced, and the labor of maintenance, etc. can be reduced.

In the above construction, it is desirable to adjust the concentration of active oxygen species (hypochlorous acid) in electrolytic water to a target concentration. The target concentration is normally set to a value enough to inactivate microorganisms such as virus, bacteria, fungus, etc. which frequently exist in a set-up place of the floor-mount type air filtering apparatus (for example, school). In this case, the concentration of the active oxygen specifies (for example, hypochlorous acid) in the electrolytic water can he adjusted by changing the current flowing through the electrodes 32 and 33 or the voltage applied between the electrodes 32 and 33. For example, by increasing the current flowing through the electrodes 32 and 33 (for example, 40mA/cm² in current density), the concentration of hypochlorous acid can be changed to a high value . Accordingly, the concentration of active oxygen species can be changed by merely adjusting the voltage applied to the electrodes or adjusting the current flowing through the electrodes without using any new device, and thus the cost can be reduced and the space can be saved.

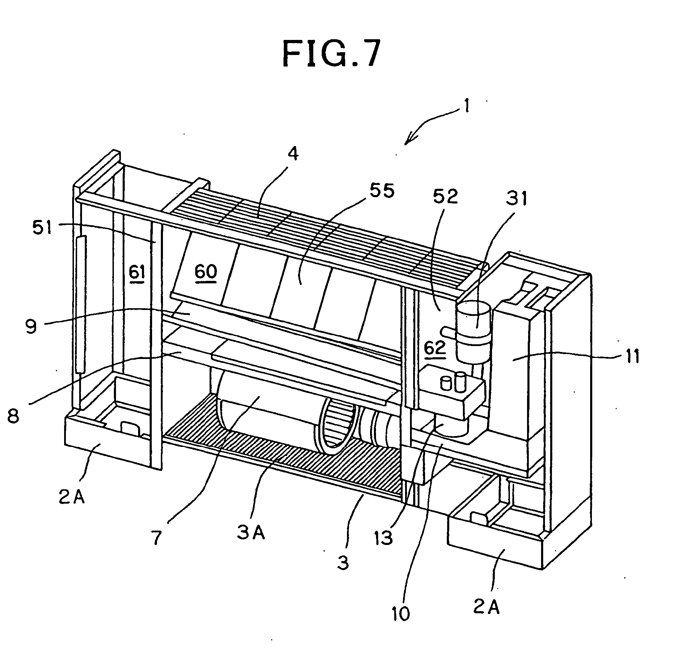

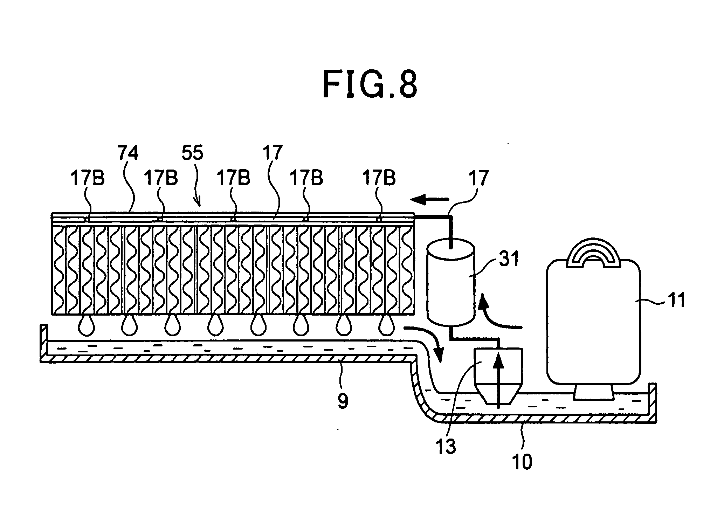

Fig. 7 shows the internal construction of the floor-mount type air filtering apparatus using another embodiment of the gas-liquid contact member 5. The gas-liquid contact member 55 shown in Fig. 7 comprises plural (for example, five) element portions E, and Fig. 8 is a diagram corresponding to Fig. 5A. In this case, the respective water spray holes 17B are formed at the positions corresponding to the respective element portions E of the gas-liquid contact member 55.

The gas-liquid contact member 55 shown in Fig. 7 will be described.

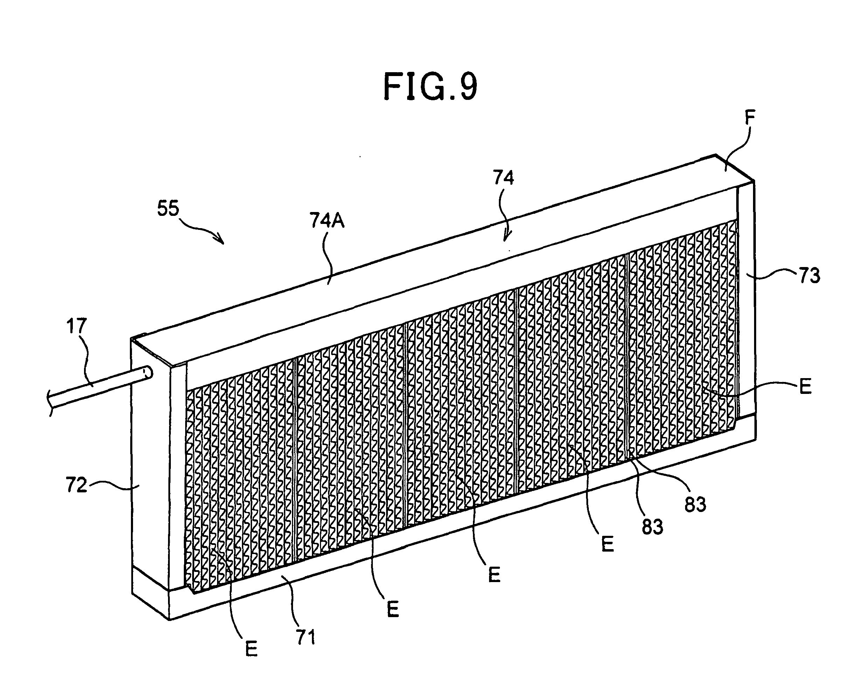

As shown in Fig. 9, the gas-liquid contact member 55 comprises plural (five in this embodiment) element portions E into which electrolytic water is infiltrated and through which air such as indoor air or the like is passed, and a frame portion F for supporting the element portions E. The frame portion F is equipped with a lower frame 71, a pair of vertical frames 72 and 73 fixed to both the ends of the lower frame 71, the electrolytic water supply pipe 17 that is disposed so as to penetrate through the upper portion of one vertical frame 72 and fixed to the other vertical frame 73 at the tip thereof, and the water spray portion 74 comprising the water spray box 74' and an upper frame fixed between the upper end portions of the pair of vertical frames 72 and 73. That is, the electrolytic water supply pipe 17 is inserted in the water spray box 74' formed along in the longitudinal direction of the gas-liquid contact member 55. The portion of the electrolytic water supply pipe 17 which is inserted in the water spray box 74' corresponds to the insertion portion 17A shown in Fig. 5A.

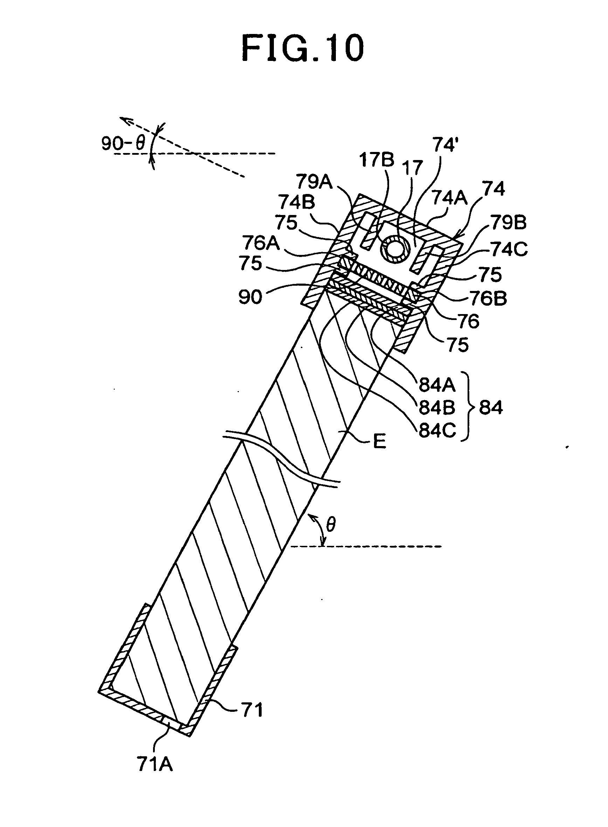

Fig. 10 shows the detailed construction of the water spray portion 74. As shown in Fig. 10, the water spray portion 74 has the upper frame comprising an upper plate 74A, a front plate 74B and a back plate 74C which are assembled so as to have substantially the U-shaped section. A pair of support members 75 extending substantially in parallel to the upper plate 74A are formed on the inner surfaces of the front plate 74B and the back plate 74C as shown in Fig. 10. Furthermore, a first water distributing sheet 76 is inserted between the support members 75 and disposed below the electrolytic water supply pipe 17. The electrolytic water dropped from the many water spray holes 17B of the electrolytic water supply pipe 17 is diffused into the first water distributing sheet 76, whereby the electrolytic water can be uniformly supplied onto the upper surfaces of the element portions E.

Furthermore, a pair of guide pieces 79A and 79B may be formed on the inner surface of the upper plate 74A so as to extend substantially in parallel to the front plate 74B and the back plate 74C. A drain port 71A is formed in the lower surface of a lower frame 71 of each element portion E to discharge surplus electrolytic water dropped to each element portion E to the water receiving tray 9. The guide pieces 79A and 79B serve as guide the insertion port 17A when the insertion portion 17A is inserted into the water spray box 74' . As described later, the guide piece 79A also enables electrolytic water to be uniformly infiltrated into the gas-liquid contact member.

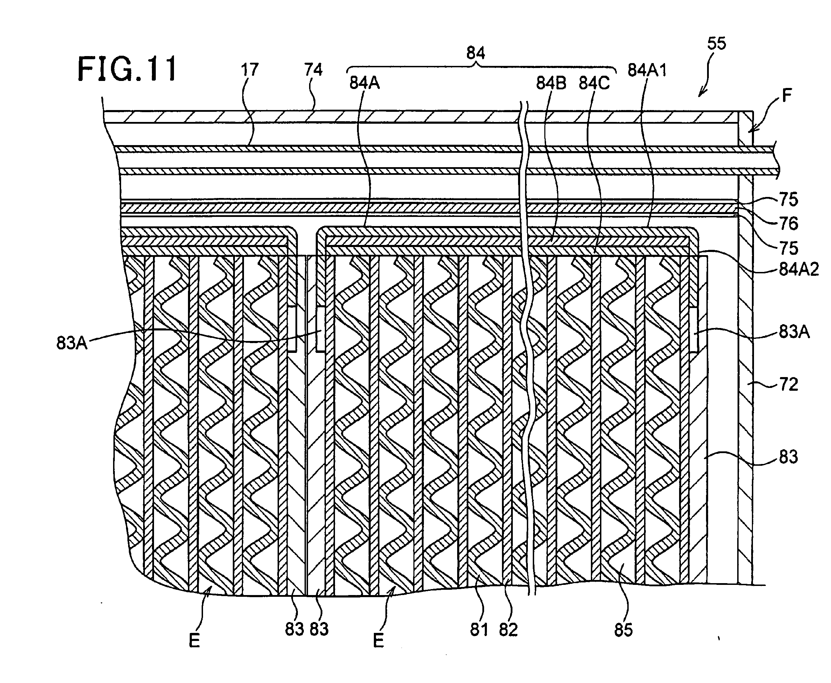

Each element portion E is formed by laminating corrugated wave-shaped members 81 and flat-plate type members 82 as shown in Fig. 11 and arranging a pair of frame members 83 constituting a vertical frame for the element portion E concerned at both the ends of the laminated corrugated members 81 and flat-plate members 82. The frame members 83 are arranged at both the ends of the laminated assembly of the corrugated members 81 and flat-plate members 82, whereby the element portion E concerned can be easily handled. Furthermore, the element portion E is constructed by laminating the corrugated members 81 and flat-plate members 82, and thus a large number of substantially triangular openings 85 are formed between the corrugated member 81 and the flat-plate member 82. Therefore, there can be achieved such a structure that the gas-liquid contact area can be broadly secured, the electrolytic water can be dropped and clogging hardly occurs.

The respective element portions E are arranged between the vertical frames 72 and 73 of the frame portion F, and plural second water distributing sheets 84 are arranged on the upper surfaces (upper edge portions) of the element portions E. The second distributing sheets 84 serve to diffuse the electrolytic water dropped to the upper surfaces of the respective element portions E through the first water distributing sheet 76 onto the whole upper surfaces of the element portions E.

In this embodiment, the second distributing sheet 84 comprises three distributing sheets (an upper distributing sheet 84A, an intermediate distributing sheet 84B and a lower distributing sheet 84C). The length of the intermediate distributing sheet 84B and the lower distributing sheet 84C is set to be substantially equal to the length in the width direction of the assembly of the laminated corrugated members 81 and the flat-plate members 82 of each element portion E. The upper distributing sheet 84A comprises a base portion 84A1 having substantially the same length as the intermediate distributing sheet 84B and the lower distributing sheet 84C, and bent portions 84A2 provided to both the ends of the base portion 84A1. By inserting the bent portions 84A2 into an insertion port 83A formed in the frame member 83, the three distributing sheets 84A to 84C can be stacked and fixed to the upper surface of the element portion E.

The corrugated members 81, the flat-plate members 82, the first distributing sheet 76 and the second distributing sheet 84 are formed of materials which have liquid permeability and are hardly deteriorated by electrolytic water, for example, polyolefin type resin (polyethylene resin, polypropylene resin or the like), PET (Polyethylene terephthalate) resin, vinyl chloride resin, fluorocarbon polymer (PTFE, PFA, ETFE or the like), ceramic type material or the like. In this construction, PET resin is used. Electrolytic water has an antirust property, and thus it is unnecessary to coat antirust agent to the gas-liquid contact member 55.

As described above, the gas-liquid contact member 55 is designed in a rectangular shape, and it is disposed so as to be tilted (inclined) in the housing 2. Therefore, when electrolytic water is simply dropped, the electrolytic water concentrates on the lower portion 76B of the first distributing sheet 76. Therefore, the electrolytic water infiltrates to the back surface side of the element portion E, however, the electrolytic water hardly infiltrates to the front surface side of the element portion E, so that the electrolytic water cannot uniformly infiltrate to the element portion E and thus a sufficient air filtering effect cannot be achieved.

Therefore, according to this embodiment, in order to uniformly supply electrolytic water to the whole surface of the first distributing sheet 76, the water spray holes 17B are formed in the electrolytic water supply pipe 17 so as to face substantially sideways to the front surface side of the housing 2. In this specification, "facing sideways" contains not only "facing in the horizontal direction", but also "facing in any direction of any angle along at which electrolytic water can be uniformly supplied to the first distributing sheet 76 or the upper surface of the element portion E.

Specifically, as shown in Fig. 10, the water spray holes 17B are so as to face the guide piece (inner wall) 79A formed at the front plate 74B side of the upper frame 74, and when the inclination angle of the gas-liquid contact member 55 is represented by θ (degree), the water spray holes 17B are formed at the positions at which the elevation angle with respect to the horizontal plane is equal to 90-θ (degree). Furthermore, the water spray holes 17B are formed on a substantially straight line in the axial direction of the electrolytic water supply pipe 17 so as to be spaced from one another at a predetermined interval.

According to this construction, the electrolytic water discharged from the water spray holes 17B of the electrolytic water supply pipe 17 impinges against the guide piece 79A formed on the inner surface of the upper frame 74 to be dropped to the upper portion 76A side of the first distributing sheet 76. The dropped electrolytic water moves from the upper portion 76A side of the first distributing sheet 76 to the lower portion 76B side along the slope of the first distributing sheet 76, whereby the electrolytic water infiltrates and diffused into the whole area of the first distributing sheet 76. The electrolytic water concerned is dropped onto the second distributing sheet 84 and diffused into the whole second distributing sheet 84, so that the electrolytic water infiltrates into the whole upper portion of the element portion E and then infiltrates downwardly along the slope of the gas-liquid contact member 55 Accordingly, the electrolytic water can be uniformly infiltrated into the whole area of the gas-liquid contact member 55. Therefore, breeding of various microorganisms in the gas-liquid contact member 55 can be prevented, and virus floating in the air and passing through the gas-liquid contact member 55 can be inactivated.

Furthermore, the total amount of water sprayed from the many water spray holes 17B (the total sprayed water amount corresponding to the total hole diameter) is set to be smaller than the discharge amount of the circulating pump 13. Accordingly, according to this construction, the pressure of electrolytic water in the insertion portion 17A of the electrolytic water supply pipe 17 is increased, so that the electrolytic water is sufficiently supplied to all the water spray holes 17B from end to end. Accordingly, the electrolytic water can be evenly infiltrated to the gas-liquid contact member 55 from end to end in the longitudinal direction of the gas-liquid contact member 55.

Next, the operation of the air filtering apparatus of the floor-mount type air filtering apparatus having the gas-liquid contact member 55 of Fig. 9 will be described.

In Fig. 1, when the operation lid 2D is opened, the operation panel 41 is exposed (see Fig. 2). By operating the operation panel 41, the floor-mount type air filtering apparatus 1 starts to operate. When the operation is started, the circulating pump 13 is driven, and water such as tape water or the like stocked in the water supply tank support tray 10 is supplied to the electrolytic bath 31 as shown in Fig. 8.

In this electrolytic bath 31, the water is electrolyzed by supplying current to the electrodes 32 and 33 to generate electrolytic water containing active oxygen species. The electrolytic water concerned is discharged from the water spray holes 17B (Fig. 10) of the electrolytic water supply pipe 17, impinges against the guide piece 79A of the upper frame 74 and then drops onto the first distributing sheet 76. Accordingly, the electrolytic water diffuses into the whole area of the first distributing sheet 76, and the electrolytic water concerned drops onto the second distributing sheet 84. The dropped electrolytic water infiltrates into the second distributing sheet 84, and diffused into the whole area of the second distributing sheet 84, so that the electrolytic water is supplied to the whole area of the upper portion of the element portion E. Accordingly, the electrolytic water infiltrates to the corrugated members 81 and the flat-type members 82 of the element portion E from end to end.

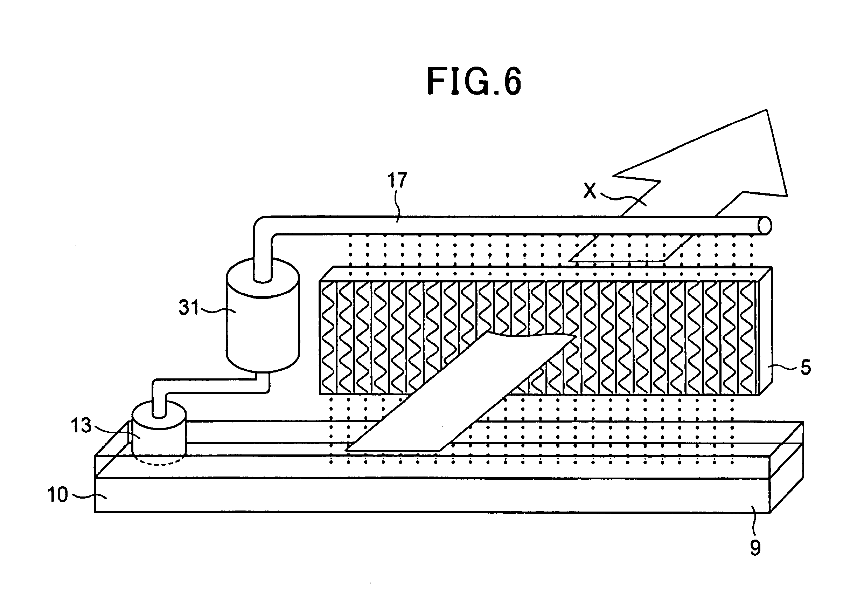

The electrolytic water dropped from the gas-liquid contact member 5 is drained to the water receiving tray 9 through a drain outlet 71A formed in the lower frame 71 (Fig. 6) . The electrolytic water flows along the downwardly inclined water receiving tray 9 into the support tray 10, and is stocked there. This embodiment adopts a water circulating system, and thus when the amount of water is reduced due to vaporization or the like, a suitable amount of water such as tap water in the water supply tank 11 is supplied to the support tray 10. This water supply tank 11 is disposed so as to be freely detachable by opening the opening/closing lid 2E, and the water supply tank 11 can be taken out and supplemented with water such as tap water.

Indoor air is supplied through the air blowing fan 7 to the gas-liquid contact member 55 into which the electrolytic water infiltrates. The indoor air is brought into contact with the active oxygen specifies contained in the electrolytic water infiltrating into the element portions E of the gas-liquid contact member 55 and then blown out to the room again. For example, when influenza virus inducing infection is floated in the indoor air, the active oxygen species has a function of breaking and vanishing (removing) the surface proteins of the virus. When influenza virus is broken, the influenza virus is not bonded to a receptor which is required for the influenza virus to infect, and thus the infection can be prevented. As a demonstration test result, it has been found that when air having influenza virus floated therein is passed through the gas-liquid contact member 5 of this embodiment, 99% or more of influenza virus can be removed (inactivated).

According to this embodiment, the rectangular gas-liquid contact member 55 is disposed in the housing 2 so as to be tilted, electrolytic water is dropped from the upper side through the electrolytic water supply pipe 17 onto the upper surface of the element portion E of the gas-liquid contact member 55, air is blown to the element portion E in which the electrolytic water infiltrates, and then the air is blown out to the room. The water spray holes 17B which are arranged so as to face substantially sideways (substantially in the horizontal direction) so that the electrolytic water can be uniformly supplied to the upper surface of the element portion E. Therefore, the electrolytic water discharged from the water spray holes 17B is substantially uniformly supplied to the upper surface of the element portion E, and thus the electrolytic water can be substantially uniformly infiltrated in the whole area of the element portion E. Accordingly, when air is passed through the element portion E, the air is kept to be more easily brought into contact with electrolytic water, so that the air filtering efficiency of the air passing through the element portion E concerned can be enhanced.

Furthermore, according to this embodiment, the electrolytic water supply pipe 17 is inserted into the upper frame 74 formed along in the longitudinal direction of the gas-liquid contact member 55, and the water spray holes 17B are formed so as to face the guide piece 798 of the upper frame 74. Therefore, electrolytic water impinging against the guide piece 79 is dropped to the upper portion 76A side of the first distributing sheet 76. Therefore, the electrolytic water is diffused into the whole area of the first distributing sheet 76, and dropped to the second distributing sheet 84 to infiltrate into the whole area of the second distributing sheet 84. Then, the electrolytic water infiltrates into the whole upper surface of the element portion E, and then infiltrates downwardly along the slope of the gas-liquid contact member 55. Accordingly, the electrolytic water can be substantially uniformly infiltrated into the whole area of the element portion E, and the air filtering efficiency of air passing through the element portion E can be enhanced.

Still furthermore, according to this embodiment, the water spray holes 17B are arranged so as to be spaced substantially at the equal interval in the axial direction of the electrolytic water supply pipe 17. therefore, the electrolytic water dropped from the water sprayholes 77 is dropped onto the first distributing sheet 76 disposed below the electrolytic water supply pipe 17, and infiltrates into the first distributing sheet 76, whereby the electrolytic water can be substantially uniformly diffused in the longitudinal direction of the first distributing sheet 76. Accordingly, the electrolytic water can be substantially uniformly supplied to the upper surface portion of the element portions E of the gas-liquid contact member 55, and the electrolytic water can be infiltrated into the whole area of the element portions E, whereby the air filtering efficiency of air passing through the element portions e can be further enhanced.

The present invention is not limited to the above embodiment, and various modifications may be made without departing from the subject matter of the present invention described by the appended claims. For example, ozone (O₃), hydrogen peroxide (H₂O₂) or the like may be generated as active oxygen specifies. In this case, when platinum tantalum electrodes are used as the electrodes 32 and 33, active oxygen specifies can be highly efficiently and stably generated from water containing diluted ion specifies by electrolysis.

At this time, at the anode, the following reaction occurs:

2H₂O → 4H⁺ + O₂ + 4e⁻

Simultaneously with the above reaction, the following reactions occur, and ozone (O₃) is generated.

3H₂O → O₃ + 6H⁺ +6e⁻

2H₂O → O₃ + 4H⁺ + 4e-

Furthermore, at the cathode, the following reactions occur:

4H⁺ + 4e⁻ + (4OH⁻) → 2H₂ + (4OH⁻)

O₂⁻ + e⁻ + 2H⁺ → H₂O₂

That is, O₂⁻ generated through the electrode reaction and H⁺ in solution are bonded to each other to generate hydrogen peroxide (H₂O₂).

In this construction, ozone (O₃) and hydrogen peroxide (H₂O₂) which have strong sterilizing power are generated by supplying current to the electrodes, and electrolytic water containing ozone (O₃) and hydrogen peroxide (H₂O₂) can be created. The concentration of ozone or hydrogen peroxide in the electrolytic water is adjusted to a value suitable for inactivate target virus or the like and air is passed through the gas-liquid contact member 5 supplied with the electrolytic water having this concentration, whereby target virus, etc. floating in the air can be inactivated. Furthermore, even odor reacts with ozone or hydrogen peroxide in the electrolytic water when passing through the gas-liquid contact member 5, and ionized and dissolved in the electrolytic water, whereby the odor components are removed from the air and thus the air is deodorized.

When scale is deposited on the electrode (cathode) due to electrolysis of tap water, it is difficult to continue the electrolysis because the electrical conductivity is lowered.

In this case, it is effective to invert the polarities of the electrodes (switching the plus and minus polarities of the electrodes). The scale deposited on the cathode can be removed by performing electrolysis while the cathode is used as the anode. Under the polarity inverting control, the polarities of the electrodes may be inverted periodically by using a timer, or the polarities of the electrodes may be inverted irregularly, for example, every time the operation of the apparatus is started or the like. Furthermore, increase of the electrolysis resistance (reduction in electrolysis current or increase in electrolysis voltage) may be detected, and the polarities of the electrodes may be inverted on the basis of the detection result.

The above embodiment adopts the water supply system based on the water supply tank 11 which can be freely put in and out. However, the present invention may adopt a water pipe water supply system in which a tap water pipe is connected to the apparatus in place of the water supply tank 11 and city water (tap water) is introduced.

Still furthermore, in the above embodiment, the upper frame 74 is provided with the guide piece 79 which each water spray hole 17B faces. However, it is not necessarily required to provide the guide piece 79A. When no guide piece 79A is provided, the front plate 74B of the upper frame 74 serves as the guide piece 79A. Furthermore, the guide piece 79A is disposed so as to face each water spray hole 17B. In this case, a single slender guide piece 79A may be continuously formed on the inner surface of the upper frame 74A so as to extend in the longitudinal direction of the upper frame 74A and also face each water spray holes 17B, or a plurality of guide pieces 79A may be formed on the inner surface of the upper frame 74A so as to be intermittently arranged in the longitudinal direction of the upper frame 74A and face each water spray hole 17B.

Still furthermore, each element portion E is designed so that the second distributing sheet 84 is constructed by stacking the three sheets 84A to 84C, however, the present invention is not limited to this embodiment. The number of stacked sheets is not limited to three, and it may be set to any number. For example, when the flow amount of air passing through the gas-liquid contact member is largest at the center portion of the gas-liquid contact member and it is gradually reduced as the position shifts to both the end sides of the gas-liquid contact member, only one sheet is disposed at the center element portion E where the air flow amount is largest, and the number of sheets is increased like two, three, etc. as the position shifts to both the end sides of the gas-liquid contact member. According to this construction, the water flow resistance at the center portion is reduced, and thus the supply amount of electrolytic water at the center portion at which the air flow amount is large is increased. Accordingly, the electrolytic water whose amount corresponds to the air flow amount canbe supplied to the gas-liquid contact member, whereby virus, etc. floating in the air passing through the gas-liquid contact member can be effectively inactivated.

Still furthermore, not only the number of sheets of the second distributing sheet is changed, but also the point diameter (mesh diameter) of the second distributing sheet may be changed (specifically, the point diameter at the center portion is set to a large value, and it is reduced as the position shifts to both the end sides).

Still furthermore, the above-described embodiments adopt an indoor air circulating system in which indoor air is sucked from a room, subjected to sterile filtration (air filtering treatment) and then returned to the room. However, air to be subjected to sterile filtration is not limited to indoor air, and outdoor air may be directly subjected to sterile filtration and then supplied to the room. Furthermore, both indoor air and outdoor air may be subjected to sterile filtration, and then supplied to the room. That is, any air in-take manner may be adopted.

Still furthermore, the above-described embodiments, the air filtering apparatus is a floor-mount type air filtering apparatus that is used under the state that it is put on the floor. However, the air filtering apparatus of the present invention is not limited to the floor-mount type air filtering apparatus, and it may be used in any condition, for example, in a wall-suspended fashion, in a ceiling-suspended fashion, in a ceiling-cassette fashion or the like.

## Claims

1. An air filtering apparatus, comprising: a housing (2); at least one partition plate (51, 52) for partitioning the inside of the housing into at least two chambers (60, 61, 62); a gas-liquid contact member having water retentivity (5, 55) and equipped with a water spray portion (74) to which electrolytic water is supplied; an air blowing fan (7) for blowing air to the gas-liquid contact member so that the air is brought into contact with the electrolytic water flowing along the gas-liquid contact member; a support tray (10) for receiving electrolytic water flowing out from the gas-liquid contact member; a circulating pump (13) for pumping up the electrolytic water stocked in the support tray (10); an electrolytic water generating unit (31) for electrolyzing water; and an electrolytic water supply pipe (17) connected to the water spraying portion (74) of the gas-liquid contact member to supply the gas-liquid contact member with the electrolytic water generated by the electrolytic water generating unit,
***characterized in that*** an electrolytic water circulating system through which electrolytic water is circulated is constructed by the gas-liquid contact member (5, 55), the supply tray (9), the circulating pump (13), the electrolytic water generating unit (31) and electrolytic water supply pipe (17), the electrolytic water withdrawn from the gas-liquid contact member is pumped up and supplied to the electrolytic water generating unit (31) by the circulating pump (13), and the electrolytic water generating unit is disposed in proximity to the gas-liquid contact member (5).

2. The air filtering apparatus according to claim 1, wherein the electrolytic water supply pipe is inserted in the water spray portion (74) of the gas-liquid contact member, and plural water spray holes (17B) are formed in the electrolytic water supply pipe so as to be spaced from one another substantially at equal intervals.

3. The air filtering apparatus according to claim 1, wherein the water discharge amount of the circulating pump is set to be larger than the water spray amount from the electrolytic water supply pipe.

4. The air filtering apparatus according to claim 1, wherein the electrolytic water generated by the electrolytic water generating unit is achieved by supplying current to electrodes and electrolyzing tap water and contains active oxygen species.

5. The air filtering apparatus according to claim 4, wherein the active oxygen specifies contain at least one material of hypochlorous acid, ozone and hydrogen peroxide.

6. The air filtering apparatus according to claim 5, wherein the gas-liquid contact member is designed in a rectangular shape and disposed so as to be tilted in the housing, the electrolytic water is dropped from the upper side through the electrolytic water supply pipe to the upper edge portion of the tilted gas-liquid contact member, and air is blown to the gas-liquid contact member in which the electrolytic water infiltrates, and then blown out to a room.

7. The air filtering apparatus according to claim 6, wherein the water spray holes formed in the electrolytic water supply pipe are formed so as to face substantially sideways so that the electrolytic water can be substantially uniformly supplied to the upper edge portion.

8. The air filtering apparatus according to claim 6, wherein the electrolytic water supply pipe is inserted into the water spray portion (74) formed in the longitudinal direction of the gas-liquid contact member, and the water spray holes are formed so as to face the inner wall of the water spray portion.

9. The air filtering apparatus according to claim 6, wherein the water spray holes are formed in the electrolytic water supply pipe so as to be spaced from one another substantially at equal intervals along the axial direction of the electrolytic water supply pipe.

10. The air filtering apparatus according to claim 6, wherein the water spray portion is equipped with a distributing sheet for uniformly diffusing the electrolytic water dropped from the water spray holes, thereby supplying the electrolytic water to the upper end portion of the gas-liquid contact member uniformly.

11. The air filtering apparatus according to claim 10, wherein a guide member (79A) disposed so as to face each water spray hole and guide electrolytic water discharged from the water spray hole concerned to the distributing sheet is provided in the water spray portion.

12. The air filtering apparatus according to claim 1, wherein the air blowing fan (7) and the gas-liquid contact member (5, 55) are accommodated in one chamber (60) of the at least two chambers, and the support tray (10), the circulating pump (13), the electrolytic water generating unit (31) and the electrolytic water supply pipe (17) are accommodated in the other chamber (62).

## Patentansprüche

1. Luftfilterungsvorrichtung mit: einem Gehäuse (2); mindestens einer Trennwand (51, 52) zum Tennen des Inneren des Gehäuses in mindestens zwei Kammern (60, 61, 62); einem Gas-Flüssigkeits-Kontaktelement (5, 55), das Wasserrückhaltevermögen aufweist und mit einem Wassersprühteil (74) ausgerüstet ist, dem elektrolytisches Wasser zugeführt wird; einem Luftgebläseventilator (7) zum Blasen von Luft auf das Gas-Flüssigkeits-Kontaktelement, sodass die Luft in Kontakt mit dem elektrolytischen Wasser gebracht wird, das entlang des Gas-Flüssigkeits-Kontaktelements fließt; einer Trägerwanne (10) zum Aufnehmen des elektrolytischen Wassers, das aus dem Gas-Flüssigkeits-Kontaktelement heraus fließt; einer Umwälzpumpe (13) zum Hochpumpen des elektrolytischen Wassers, das in der Trägerwanne (10) gelagert wird; einer Erzeugungseinheit (31) für elektrolytisches Wasser zum Elektrolysieren von Wasser; und einer Zufuhrleitung (17) für elektrolytisches Wasser, die mit dem Wassersprühteil (74) des Gas-Flüssigkeits-Kontaktelements verbunden ist, um das Gas-Flüssigkeits-Kontaktelement mit dem elektrolytischen Wasser zu versorgen, das durch die Erzeugungseinheit für elektrolytisches Wasser erzeugt wird,
**dadurch gekennzeichnet, dass** ein Zirkulationssystem für elektrolytisches Wasser, durch das elektrolytisches Wasser umgewälzt wird, durch das Gas-Flüssigkeits-Kontaktelement (5, 55), die Zufuhrwanne (9), die Umwälzpumpe (13), die Erzeugungseinheit für elektrolytisches Wasser (31) und die Zufuhrleitung für elektrolytisches Wasser (17) gebildet wird, wobei das elektrolytische Wasser, das aus dem Gas-Flüssigkeits-Kontaktelement abgezogen wird hoch gepumpt wird und der Erzeugungseinheit für elektrolytisches Wasser (31) durch die Umwälzpumpe (13) zugeführt wird und die Erzeugungseinheit für elektrolytisches Wasser in der Nähe des Gas-Flüssigkeits-Kontaktelements (5) angeordnet ist.

2. Luftfilterungsvorrichtung gemäß Anspruch 1, wobei die Zufuhrleitung für elektrolytisches Wasser in dem Wassersprühteil (74) des Gas-Flüssigkeits-Kontaktelements eingeführt ist und mehrere Wassersprühlöcher (17B) in der Zufuhrleitung für elektrolytisches Wasser gebildet sind, sodass sie voneinander im Wesentlichen in gleichen Abständen beabstandet sind.

3. Luftfilterungsvorrichtung gemäß Anspruch 1, wobei die Wasserausstoßmenge der Umwälzpumpe so eingestellt ist, dass sie größer ist als die Wassersprühmenge aus der Zufuhrleitung für elektrolytisches Wasser.

4. Luftfilterungsvorrichtung gemäß Anspruch 1, wobei das elektrolytische Wasser, das von der Erzeugungseinheit für elektrolytisches Wasser erzeugt wird, durch das Zuführen von Strom zu Elektroden und das Elektrolysieren von Leitungswasser erhalten wird und aktive Sauerstoffspezies enthält.

5. Luftfilterungsvorrichtung gemäß Anspruch 4, wobei die aktiven Sauerstoffspezies mindestens ein Material aus hypochloriger Säure, Ozon und Wasserstoffperoxid enthalten.

6. Luftfilterungsvorrichtung gemäß Anspruch 5, wobei das Gas-Flüssigkeits-Kontaktelement in einer rechteckigen Form entworfen ist und so angeordnet ist, dass es in dem Gehäuse geneigt ist, das elektrolytische Wasser von der Oberseite durch die Zufuhrleitung für elektrolytisches Wasser zu dem oberen Randbereich des geneigten Gas-Flüssigkeits-Kontaktelements getropft wird und Luft zu dem Gas-Flüssigkeits-Kontaktelement geblasen wird, in welches das elektrolytische Wasser eindringt, und dann zu einem Raum herausgeblasen wird.

7. Luftfilterungsvorrichtung gemäß Anspruch 6, wobei die Wassersprühlöcher, die in der Zufuhrleitung für elektrolytisches Wasser gebildet sind, so gebildet sind, dass sie im Wesentlichen seitwärts gerichtet sind, sodass das elektrolytische Wasser im Wesentlichen gleichförmig dem oberen Kantenbereich zugeführt werden kann.

8. Luftfilterungsvorrichtung gemäß Anspruch 6, wobei die Zufuhrleitung für elektrolytisches Wasser in den Wassersprühteil (74), der in der Längsrichtung des Gas-Flüssigkeits-Kontaktelements gebildet ist, eingeführt ist und die Wassersprühlöcher so gebildet sind, dass sie der Innenwand des Wassersprühteils gegenüberliegen.

9. Luftfilterungsvorrichtung gemäß Anspruch 6, wobei die Wassersprühlöcher in der Zufuhrleitung für elektrolytisches Wasser so gebildet sind, dass sie voneinander im Wesentlichen in gleichen Abständen entlang der Axialrichtung der Zufuhrleitung für elektrolytisches Wasser beabstandet sind.

10. Luftfilterungsvorrichtung gemäß Anspruch 6, wobei der Wassersprühteil mit einem Verteilungsblatt zum gleichförmigen Verteilen des elektrolytischen Wassers, das aus den Wassersprühlöchern heraustropft, ausgestattet ist, sodass das elektrolytische Wasser gleichförmig dem oberen Endteil des Gas-Flüssigkeits-Kontaktelements zugeführt wird.

11. Luftfilterungsvorrichtung gemäß Anspruch 10, wobei ein Führungselement (79A) so angeordnet ist, dass es jedem Wassersprühloch gegenübersteht und elektrolytisches Wasser, das aus dem jeweiligen Wassersprühloch ausgestoßen wird, zu dem Verteilungsblatt führt, das in dem Wassersprühteil vorgesehen ist.

12. Luftfilterungsvorrichtung gemäß Anspruch 1, wobei der Luftgebläseventilator (7) und das Gas-Flüssigkeits-Kontaktelement (5, 55) in einer Kammer (60) der mindestens zwei Kammern aufgenommen sind, und die Trägerwanne (10), die Umwälzpumpe (13), die Erzeugungseinheit für elektrolytisches Wasser (31) und die Zufuhrleitung für elektrolytisches Wasser (17) in der anderen Kammer (62) aufgenommen sind.

## Revendications

1. Dispositif de filtrage d'air, comprenant : un boîtier (2) ; au moins une première plaque de séparation (51, 52) destinée à diviser l'intérieur du boîtier en au moins deux chambres (60, 61, 62) ; un élément de contact gaz-liquide présentant une certaine capacité de rétention d'eau (5, 55) et équipé d'une partie de pulvérisation d'eau (74) à laquelle de l'eau électrolytique est délivrée ; un ventilateur de soufflage d'air (7) destiné à souffler de l'air dans l'élément de contact gaz-liquide de telle sorte que l'air est amené en contact avec l'eau électrolytique circulant le long de l'élément de contact gaz-liquide ; un plateau support (10) destiné à recevoir l'eau électrolytique s'écoulant à partir de l'élément de contact gaz-liquide ; une pompe de circulation (13) destinée à pomper l'eau électrolytique stockée sur le plateau support (10) ; une unité de production d'eau électrolytique (31) destinée à électrolyser l'eau ; et une tuyauterie d'alimentation en eau électrolytique (17) raccordée à la partie de pulvérisation d'eau (74) de l'élément de contact gaz-liquide afin d'alimenter l'élément de contact gaz-liquide avec l'eau électrolytique produite par l'unité de production d'eau électrolytique,
**caractérisé en ce qu'**un dispositif de mise en circulation d'eau électrolytique par l'intermédiaire duquel de l'eau électrolytique est mise en circulation est construit par l'élément de contact gaz-liquide (5, 55), le plateau d'alimentation (9), la pompe de circulation (13), l'unité de production d'eau électrolytique (31) et la tuyauterie d'alimentation en eau électrolytique (17), l'eau électrolytique extraite de l'élément de contact gaz-liquide est pompée et délivrée à l'unité de production d'eau électrolytique (31) par la pompe de circulation (13), et l'unité de production d'eau électrolytique est disposée à proximité de l'élément de contact gaz-liquide (5).

2. Dispositif de filtrage d'air selon la revendication 1, dans lequel la tuyauterie d'alimentation en eau électrolytique est insérée dans la partie de pulvérisation d'eau (74) de l'élément de contact gaz-liquide, et plusieurs orifices de pulvérisation d'eau (17B) sont formés sur la tuyauterie d'alimentation en eau électrolytique de manière à être espacés les uns des autres à intervalles sensiblement réguliers.

3. Dispositif de filtrage d'air selon la revendication 1, dans lequel le débit d'eau de sortie de la pompe de circulation est défini de manière à être supérieur au débit de pulvérisation d'eau par la tuyauterie d'alimentation en eau électrolytique.

4. Dispositif de filtrage d'air selon la revendication 1, dans lequel l'eau électrolytique produite par l'unité de production d'eau électrolytique est obtenue en délivrant un courant sur des électrodes et en électrolysant de l'eau du robinet et elle contient des espèces à base d'oxygène actif.

5. Dispositif de filtrage d'air selon la revendication 4, dans lequel les espèces à base d'oxygène actif contiennent au moins un matériau parmi l'acide hypochloreux, l'ozone et le peroxyde d'hydrogène.

6. Dispositif de filtrage d'air selon la revendication 5, dans lequel l'élément de contact gaz-liquide est défini d'une forme rectangulaire et est disposé de manière à être basculé dans le boîtier, l'eau électrolytique est écoulée à partir du côté supérieur par l'intermédiaire de la tuyauterie d'alimentation en eau électrolytique vers la partie de bord supérieure de l'élément de contact gaz-liquide basculé, et de l'air est soufflé vers l'élément de contact gaz-liquide dans lequel l'eau électrolytique s'infiltre, et soufflé ensuite vers une salle.

7. Dispositif de filtrage d'air selon la revendication 6, dans lequel les orifices de pulvérisation d'eau formés sur la tuyauterie d'alimentation en eau électrolytique sont formés de manière à faire sensiblement face aux côtés de telle sorte que l'eau électrolytique peut être délivrée sensiblement uniformément à la partie de bord supérieure.

8. Dispositif de filtrage d'air selon la revendication 6, dans lequel la tuyauterie d'alimentation en eau électrolytique est insérée dans la partie de pulvérisation d'eau (74) formée dans la direction longitudinale de l'élément de contact gaz-liquide, et les orifices de pulvérisation d'eau sont formés de manière à faire face à la paroi interne de la partie de pulvérisation d'eau.

9. Dispositif de filtrage d'air selon la revendication 6, dans lequel les orifices de pulvérisation d'eau sont formés sur la tuyauterie d'alimentation en eau électrolytique de manière à être espacés les uns des autres à intervalles sensiblement réguliers suivant la direction axiale de la tuyauterie d'alimentation en eau électrolytique.

10. Dispositif de filtrage d'air selon la revendication 6, dans lequel la partie de pulvérisation d'eau est équipée d'une plaque de distribution afin de diffuser uniformément l'eau électrolytique écoulée à partir des orifices de pulvérisation d'eau, délivrant ainsi l'eau électrolytique de manière uniforme sur la partie d'extrémité supérieure de l'élément de contact gaz-liquide.

11. Dispositif de filtrage d'air selon la revendication 10, dans lequel un élément de guidage (79A) disposé de manière à faire face à chaque orifice de pulvérisation d'eau et à guider l'eau électrolytique écoulée à partir de l'orifice de pulvérisation d'eau concerné vers la plaque de distribution est formé sur la partie de pulvérisation d'eau.

12. Dispositif de filtrage d'air selon la revendication 1, dans lequel le ventilateur de soufflage d'air (7) et l'élément de contact gaz-liquide (5, 55) sont agencés dans une première chambre (60) parmi les au moins deux chambres, et le plateau support (10), la pompe de circulation (13), l'unité de production d'eau électrolytique (31) et la tuyauterie d'alimentation en eau électrolytique (17) sont agencés dans l'autre chambre (62).
